# EUROPEAN PATENT SPECIFICATION

(11) **EP 0 927 552 B1**
(45) Date of publication and mention of the grant of the patent: **10.09.2003**
(21) Application number: 98100079.7
(22) Date of filing: 05.01.1998
(51) Int. Cl.: A61J 1/10, A61L 2/26

(54) **A flexible collapsible blood bag**
Flexibler, zusammenfaltbarer Blutbeutel
Poche de sang flexible et repliable

(43) Date of publication of application: 07.07.1999
(73) Proprietor: Mitra Industries Limited, New Delhi 110020 (IN)
(72) Inventor: Mahajan, Nitin, Worli, Mumbai-400025 (IN)
(74) Representative: WILHELMS, KILIAN & PARTNER Patentanwälte

(56) References cited:
- EP-A- 0 537 863
- EP-A- 0 778 030
- FR-A- 2 534 477
- US-A- 3 205 889
- US-A- 4 194 622
- US-A- 5 494 196

## Description

The present invention relates to a flexible collapsible blood bag as it is mentioned in the preamble of claim 1. A blood bag of this type is known from the FR-A 2 534 477.

### BACKGROUND OF THE INVENTION

Parenteral solutions are kept in plastic containers. Specifically it is known to house blood components in flexible containers. These containers referred to as blood bags can be used to receive a blood component, process the blood component, store the blood component, and assist in infusing the blood component as well in infusing the blood component into a recipient.

The desirable features of a container which contains life saving blood and blood components are that the design of the plastic container intended to be used as a blood bag shall be such that it provide for safe and convenient collection, storage, processing, transport, separation and administration of blood and blood components. Further design and manufacture shall not adversely affect the preservation of blood and blood components. The container shall permit the preparation of plasma or centrifuged or resuspended cellular components with a minimal hazard of contamination by microorganisms. The container shall be functionally compatible with the transfusion set. Its design shall also ensure that it can be used in a centrifuge cup. These constitute some of the basic requirements of a container used for the preservation and storage of blood.

There are containers available in the market for the storage of blood. These containers are made of polyvinyl chloride and are provided with a notch at the end for vertically hanging the blood bag down so that the blood flows easily and continuously from the blood bag.

Further blood bag and other blood transfusion devise as available presently are provided with a hub, a cannula and a needle cover. During assembly of these components the sharp edges of the cannula bevel many times touches the needle cover and in that process gets damaged resulting into excessive pain to the donor or to the patient during blood collection or transfusion as the case may be. Some of the manufacturer's offer needle cover that is open from the bevel end. If this end is not thoroughly sealed, it may lead to bacterial contamination.

The U.S. Patent 3, 064, 647 defines a blood component separation apparatus which comprises of means formed from a haemorepellant material. The apparatus as defined suffers from the problems associated with the leaking of the container. The U.S, Patent 3,187,750 defines a sterile integral unit containing a suitable anticoagulant having provision for separating a given quantity of stored blood into smaller quantities without breakage of seal or disturbing the sterility of the unit. This patent elaborates on the usage of the blood in required quantities and also defines needle assembly for the blood bag. However, the needle assembly here is not provided with any covering thereby leading to cause for infection. Similar references can be given to several U.S. patents such as 3, 746,001 which defines a specific aspect of the containers for parenteral solutions having pierceable access port. Again this patent defines a specific aspect of the containers for blood but fails to improvise on the problems pertaining to needle assembly and the sterility of the containers.

There are several practical problems encountered in the lyophilization, storage and reconstitution of fluid products, particularly blood in the conventional containers. Besides the practical problems of handling the blood and the blood components in the parenteral solution container, there are problems associated with the containers themselves. The parenteral solution containers used for the preservation and storage of blood and blood components must not only be susceptible to sterilization, but must also mechanically withstand low temperatures, particularly, for example, liquid nitrogen temperatures, used for lyophilization processes and withstand application of a vaccum.

A problem in the existing blood bags or plastic medical bags in the market is that they develop fungi growth during the period of its shelf life which many time is not noticed especially if the fungal growth is not distinctly visible or if proper care is not taken to inspect the bag prior to its use.

The parenteral solution containers used for or in the preservation of the blood bags of the conventionally known type have been known to suffer from leakage, This is mainly because of lack of proper sealing on the port tubing. This results in poor strength of the port tube sealing in terms of collapsibility and may also result in leakage. The present invention aims at overcoming the problems associated with the conventional parenteral solution containers for the purposes of storing or preserving blood.

Yet another problem being faced in the blood bags as available in the market today is the hanger notch is either not provided by many manufacturers or it is not suitably designed so that the blood bag can hang vertically down during transfusion.

Further the conventional PVC tubing as provided to the parenteral solution containers is very soft and gets crushed leading to problems in transfusion of the blood as well as in collection of blood. Therefore it is essential to find a suitable PVC tubing to overcome this problem encountered with the use of conventional blood bags.

Blood bags must provide a container that allows gas transmission through the container in order to maintain the viability of the cells to be housed therein. In this regard, it is necessary that the bag allow carbon dioxide to flow out of the blood bags and oxygen to flow therein. Paper provides a substrate that allows for a flow of gas through the labeled area of the blood bag; a paper label does not decrease the effective area of the bag that allows gas transmission beyond acceptable limits.

Further, it is also very desirable feature to provide some means for identifying certain information on the blood bag, e.g., the type of storage solution, anticoagulant, or blood component, the collection date, manufacture's product code and lot number, etc.

To this end, it is known to provide labels for such containers specially for blood bags. Typically, these labels have previously comprised a paper substrate that was secured to the parenteral solution container. These paper labels exhibit many characteristics that are necessary and/or desirable for a label stuck on a parenteral solution container specifically on a blood bag.

Paper also provides a surface that can be written or printed on. Thus, a paper label provides a substrate that allows one to easily indicate necessary information on the blood bag. In this regard, the label typically receives printed as well as hand written information. It is also known to use bar codes on such labels.

Paper, however, does exhibit certain disadvantages when used as a label for blood bag. Paper labels are not very durable to moisture, abrasion, temperature extreme, and are not elastic to allow for dimensional changes that occur to the blood bag. It is known to process the blood components stored within the blood bags by centrifuging the bag in addition to other processes. During such processes the labels can become wet and subjected to extreme temperatures. Paper labels can crack or wrinkle during such processes. The cracking or wrinkling of a label is especially detrimental to the use of bar codes on such labels. Unless a smooth uninterrupted surface is provided the bar codes may be unreadable by a bar code reader. This forces manual entering of data into a computer thereby increasing the chances for errors.

Further the present containers for parenteral solutions specifically blood bags available in the market use adhesives, including hot melt adhesives applied in the form of paste to provide pregummed labels. But these adhesives fail to give permanent labels as a result of which the labels are neither tamper proof nor permanent. These labels tend to peel off the soft PVC blood bag film during storage and many times create confusion and result in mislabelling of the blood bag when many such labels come out from the blood bag during storage of the blood bags filled with blood. Mislabelling of a blood bag can prove fatal for the blood- recipient, therefore it is essential to overcome these drawbacks.

Further the blood bags or plastic medical bags known in the art do not possess any temperature or time sensitive mark in the label which assures its sterility even by mere visual checking.

Another problem in the existing parenteral solution containers such as blood bags or plastic medical bags in the market is that they develop fungal growth during the period of its shelf life which many times may go unnoticed if the fungal growth is not distinctly visible or if proper care is taken to inspect the bag prior to its use.

### SUMMARY OF THE PRESENT INVENTION

The object of the invention is to provide for the safe and convenient collection, storage, processing, transport, separation and administration of blood and its components.

This object is attained by a blood bag as it is defined in claim 1.

The blood bag of the present invention comprises of a novel needle assembly for parenteral solution containers. The specific novel needle assembly protects the needle with the help of a cap and all the ends are thoroughly sealed so that contamination of any type can not enter the parenteral solution container.

The further feature of the invention is that the blood bag of the present invention is provided with a two rib assembly which prevents any leakage from the sides of the blood bag.

Further the blood bag of the present invention also exhibits certain more features which helps in convenient utilisation of the containers at the same time eliminating chances of contamination..

The present invention thus relates to a blood bag used for the purpose of preserving and storage of blood and more particularly to a parenteral solution container suitable for use as in direct or indirect contact with human blood, such as an artificial valve, artificial blood vessel, blood transfusion set, solution infusion set or blood. The blood bag of the present invention is a relatively soft and is stable even at sub zero temperatures.

The conservation of storage space is a practical problem. It is desirable that the blood bag in which the blood is stored takes up minimum amount of room, since in many instances it is stored in a controlled temperature environment, such as refrigerator, in which the space is limited. Therefore the present invention aims at a blood bag made from soft polyvinyl chloride which can be easily and compactly arranged. Further the strength of the conventional blood bag is also a problem which needs to be improvised upon. The composition for the formation of the soft polyvinyl plastic is a novel composition.

Many of the above noted blood bags have been made of plastic materials which meet the non-toxicity requirements. In such plastic materials, polyvinyl chloride is widely used because it is inexpensive, widely available in broad range of stiffness from flexible to rigid depending on the plasticizer content to serve for various purposes, and, moreover, is transparent and easy processing.

However, the conventional blood bags suffer from a few serious drawbacks especially pertaining to the strength of the blood bags. The conventional blood bags as available suffer from a serious drawback that it develops cracks at sub zero storage of blood and can not be autoclaved at sterilizing temperatures. The present invention aims at overcoming these drawbacks and providing a blood bag which does not develop cracks at sub zero storage of blood and is also extrudable, moldable and transparent.

Specific embodiments of the blood bag according to the present invention are subject matter of claims 2 - 15.

The blood bag of the present invention is obtained by using a novel composition for the manufacture of the parenteral solution container. The composition for this application utilizes non-toxic polyvinyl chloride resin and additives. Soft polyvinyl chloride sheeting is made by first compounding polyvinyl chloride resin with additives in a high speed heating mixer following cooling in the cooling mixer, then palletizing the above compound on palletizing plant and finally using these pellets for making final sheet/ tubing.

The composition for making soft polyvinyl chloride sheet for the said parenteral solution container comprises polyvinyl chloride resin, a phthalate plasticizer, soyabean oil, an adipate ester, a heat stabilizer, an organic phosphite chilator and a lubricant. The formulation comprises of the various components in the ratio as necessary to achieve the desired properties of the blood bag.

The polyvinyl chloride resin is taken in an amount of 100 parts by weight, the phthalate plasticizer is taken in an amount of 15-25 phr, soyabean oil accounts phr 10-15 phr, adipate is in an amount of 15-25 phr, the stabilizer that is present is in the amount of 1.5-3.0 phr, the chilator is 0.5-1.5 phr and the lubricant present is in the amount of 0.25 - 0.75 phr.

Here the phthalate plasticizer is preferably dioctyl phthalate, adipate is preferably dioctyl adipate while the stabilizer is calcium zinc heat stabilizer. The chilator that is selected is preferably an organic phosphite chilator and the lubricant used is a calcium stearate.

The composition of polyvinyl chloride resin and additives is made in a high speed heating mixer followed by cooling in a cooling mixer and then palletized on palletizing plant. The pellets thus formed are extruded through an extruder using a coat hanger die (T die) using barrel temperature between 140°-180° C and die temperature varies between 180°-190° C. The extruded sheet from the die is taken into the polishing/embossing roll track and cooled. The sheet is trimmed from the edges and slitted in the required width and wound on the winder.

The process requires maintaining the gauge/thickness in close tolerances. The web tension has also to be properly maintained using load cell as well as other web tension control devices. The soft polyvinyl chloride sheet manufactures in the said process has a shrinkage less than 10% at 120° C for about half an hour. The soft polyvinyl chloride sheet is made using a callender or any other conventional sheet manufacturing equipment.

The soft polyvinyl chloride sheet made in the present invention has a lower temperature stability and does not show any signs of crack at sub zero storage of blood or its components.

Another feature of the invention resides in the formulation used for formation of the PVC tubing. The characteristic of the formulation used for the preparation of soft PVC tubing is that it results in the formation of ideally soft PVC tubing. The soft PVC tube thus formed is neither too soft for the tube to get crushed or crumbled nor is too hard such that its handling becomes a problem. These characteristics are desirable for the tubing for a convenient collection and storage of parenteral solution in blood bags.

Soft polyvinyl chloride tubing is made by first compounding polyvinyl chloride resin with additives in a high speed heating mixer followed by cooling in the cooling mixer. In a preferred embodiment of the present invention the composition for the soft polyvinyl chloride tube for the blood bags have following formulations

| | |
|---|---|
| Polyvinyl chloride Resin | 100 parts per weight |
| Dioctyl Phthalate Plascticizer | 20-30 phr |
| Epoxidised soyabean oil | 10-15 phr |
| Dioctyl Adipate | 15-25 phr |
| Calcium Zinc heat stabilizer | 1.5-3.0 phr |
| Organic Phosphite chilator | 0.5-1.5 phr |
| Calcium Stearate | 0.25-0.75 phr |

This composition is used to make a soft polyvinyl chloride tubing ID not less than 2.5 mm and OD not less than 6.0 mm as may be required for specific use in the parenteral solution container. The tubing is made on a standard tube plant using an extruder die, cooling arrangements followed by haul off and winder.

With the blood bag of the present invention there is provided an improved needle assembly for blood storage bags or parenteral solution containers. Blood bag and other blood transfusion devices as available presently are provided with a hub, a cannula and a needle cover. During the assembly of these components the sharp edge of the cannula bevel many times touches the needle cover and in that process gets damaged resulting into excessive pain to the donor or to the patient during blood collection or transfusion as the case may be. Some of the manufacturer's offer needle cover which is open from the bevel end. If this end is not thoroughly sealed, it eventually leads to bacterial contamination.

Therefore with the blood bag of the present invention there is provided an improved needle assembly for the blood bag wherein all the drawbacks are eliminated. Therefore in one aspect of the invention there is provided a bevel protection sleeve made out of rubber for protection of the bevel, a needle cover which is closed from the bevel end and an inbuilt barrier in the needle cover.

In accordance with the present invention, rubber is used to manufacture the bevel protection sleeve. This sleeve is closed on one end and open on the another. The inner diameter of the sleeve is slightly less than the outer diameter of the cannula on which it is fitted. In addition to the bevel length, the bevel protection sleeve has additional length not less than 3 times the outer diameter of the cannula so that it can give an air tight grip on the body of the cannula next to bevel. The outer diameter of the bevel on the open end will be less than the inner diameter of the barrier provided in the needle cover. The needle cover is made from soft polyvinyl chloride by injection moulding. This will have a square or any other shape at the outside and is closed from the other end. The other end which is the open end has a inner diameter fitting to match the outer diameter of the hub where it is fitted.

The needle cover is provided with a seal which can be broken manually by twisting and with a barrier atmost at one third to one fourth of its length from the open end. The inner diameter of the inbuilt barrier is such that it prevent bevel protection sleeve from coming out with cannula.

The needle assembly is done by fixing cannula into the hub with the help of suitable adhesive. The bevel side of the cannula is then siliconised and cured for the required period of time. The bevel protection sleeve are washed ultrasonically and dried at 40°-60° C. These bevel protection sleeve are then capped on to the cannula bevel taking care that the closed tip of the bevel protection sleeve is not penetrated by the cannula bevel.

The soft polyvinyl chloride needle cap is then slid over the bevel protection sleeve once again taking care that undue pressure does not come on the sleeve so that the cannula bevel does not penetrate into the closed tip of a bevel protection sleeve. The cap is sealed air tight on to the hub with the help of adhesive making the needle assembly ready for further use.

The needle assembly for the blood bag comprises a needle cover made from soft polyvinyl chloride having its one end closed and the other end open, a hub fitted to the said needle cover, sealing means to provide air tight cover for the needle, a cannula fixed to the said hub wherein a barrier is provided near the open end of the said needle cover and a bevel protection sleeve is provided capping on to the bevel end of said cannula of the said bevel to provide protection to the sharp edge of the cannula bevel.

Another object of this invention is to improve the stability of the blood bag used for the preservation and storage of blood and blood components. A series of symmetrical notches are provided in the outer peripheral seal of the said blood bag. There are also provided hanging means in the peripheral seal of the blood bag. The said hanging means is a hanger notch in the form of tear seal provided at the bottom of the peripheral seal of the said blood bag having centrally angled hanger notch to enable the said blood bag to hang vertically during blood transfusion. The outer wall thickness of the peripheral seal around the tear seals is less than the inner wall thickness of the peripheral seal around the said tear seals. The tear seals in the said walls of the peripheral seal are preferably greater than two in number and most preferably maintained at three on each side. The hanger notch tear seal is formed during the process of the radio frequency sealing. This tear seal can be opened prior to its use for transfusion. The blood bag hangs vertically down because of the design of the hanger notch.

Further there are provided two ribs on the port tube sealing area which gives extra mechanical strength thereby eliminating any chance of collapse of port tubing. This also helps to minimize the leakage from this area.

The present invention defines two sealing ribs provided on each side of the bag on the port tubing. This is done by making suitable provisions of ribs in the dies meant for radio frequency sealing. During the process of radio frequency above ribs get sealed on both sides of the bag. These ribs act as reinforcements and eliminate any collapse of the port tubing. These ribs also help to minimize any chances of leakage from this area.

According to the present invention an improved plastic collapsible blood bag for blood and blood components made of soft polyvinyl chloride comprises a tamper proof non-peelable label, a sterility testing means by visual checking, a needle assembly connected to the said blood bag by means of soft polyvinyl chloride tubes, means for needle bevel protection, a pair of port tubes provided in the upper peripheral seal of the said blood bag wherein the pair of ribs is provided around the said port tube sealing area, to give extra strength to the blood bag eliminating chances of collapse of port tubing, a centrally located hanging means provided in the peripheral seal of the bag, a series of symmetrical tear seals provided in the outer peripheral seal of the said parenteral solution container.

The total volume of air contained in the blood collection pathway and the blood bag used for the collection of blood and for each transfer blood bag and its associated tubing is maintained at 2-5 ml.. This facilitates in easier filling in of the blood in the blood bag without introducing air in the blood bag. The blood is filled in the blood bag under negative pressure and while filling the air borne bacteria introduction is kept at a minimum by avoiding introduction of further air in the blood bag.

According to the present invention there is provided a novel non-peelable tamper proof label having a temperature or time sensitive mark. The blood bags as available in the market use adhesives applied in the form of paste to provide pregummed labels. But these adhesives, fail to give permanent labels as a result of which the labels are neither tamper proof nor permanent. These labels tend to peel off the soft polyvinyl chloride blood bag film during storage and many times may create a lot of confusion and result in mislabelling of the blood bag when many such labels come out from the bag during storage of the blood bags filled with blood. Blood bags or plastic medical bags known in the art do not possess any temperature or time sensitive mark on the label which assures its sterility even by mere visual checking.

The present invention aims at overcoming the disadvantages exhibited by the conventional labels used on parenteral solution containers such as blood bags. According to the present invention a tamperproof non-peelable label, is provided sealed to the said bag. The bag is also provided with a temperature and/or time sensitive mark to assure the sterility of each and every bag by visual check. This invention also envisages the use of specific quality of printing inks to provide non-impregnable printing on the label.

In accordance with this invention a suitable paper preferably a medical grade paper is taken for making the labels. This paper is preferably uncoated on one side with the mat surface finish. The above paper is procured in form of rolls of the suitable width.

A plastic film supported on a carrier in the form of roll having same width as that of the paper is taken. The film as used in the present invention is essentially radio frequency sealable as well as heat sealable to polyvinyl chloride.

The paper roll and the polyester film are roll mounted on a laminating and slitting machine. The uncoated mat side paper of the paper is laminated to the polyester film using temperature of 115° C-120° C on the laminating roll and using a pressure roller. The carrier film is then separated and wound on a winder.

The laminated paper is then slit into widths suitable for printing and rolls are made on winders. The laminated and slitted rolls are then taken on a multihead printing machine. The main printing contents of the labels are printed using required number of colours using only pigmented grade ink. This provides a tamperproof label which overcomes the problems associated with a paper label on a blood bag.

The subsequent printing head is used to make a mark of any suitable shape on the label using temperature and time sensitive mark. The ink used is sensitive to both temperature and time and changes its. colour only during steam autoclaving when subjected to the specific required heat at a specific temperature for the specified time. Unless the mark is subjected to the specific heat at specific temperature for the minimum specified time required for ensuring complete sterility of the contents, the temperature sensitive mark does not change its colour. This ensures a check on the sterility of the container even by a single glance on the container on which the label is pasted.

These labels are then punched into single label and stacked together and are then radio frequency sealed on the soft polyvinyl chloride film of the parenteral solution container such as blood bag or to any other medical bag.

The label's time or temperature sensitive mark changes colour when subjected to a heat treatment at 115° - 125° C for a period of at least 10 minutes during the process of steam autoclaving. The main advantages of this process is that tamperproof and non-peelable labels are formed which can be utilised on the parenteral solution container specifically blood bags. Any efforts to remove the label result only in tearing of the label and the said labels can not be peeled off under any novel circumstances of its usage.

Another advantage of the present invention is that a temperature sensitive mark is provided on the label which ensures complete sterility even by visual checking of the colour change on each container on which the label is pasted. As any undue variation during steam autoclaving results in changing the colour of the temperature and or time sensitive mark thereby ensuring the complete sterility of the contents.

Further the blood bags labelled by the above technique remain free of fungal growth provided suitable pinhole free pouching cover is provided to each bag.

Another advantage of the invention is that the pigmented ink used for printing of the label ensures non impregnation of the ink to the thickness of the film and eliminates any interference of the ink with the solution or the blood filled inside.

The plastic film used for lamination of paper for adhesion to the soft polyvinyl chloride of the bag does not give any leaching into the CPDA solution through the soft polyvinyl chloride film. The above process is user friendly and gives higher productivity.

### BRIEF DESCRIPTION OF THE ACCOMPANYING DRAWINGS

The present invention can be more clearly and precisely understood with the help of following drawings.
Figure 1 of the present invention shows the needle assembly.
Figure 2 shows the needle cover with bevel sleeve.
Figure 3 shows the parenteral solution container in its elevational view.

### DETAILED DESCRIPTION OF THE INVENTION

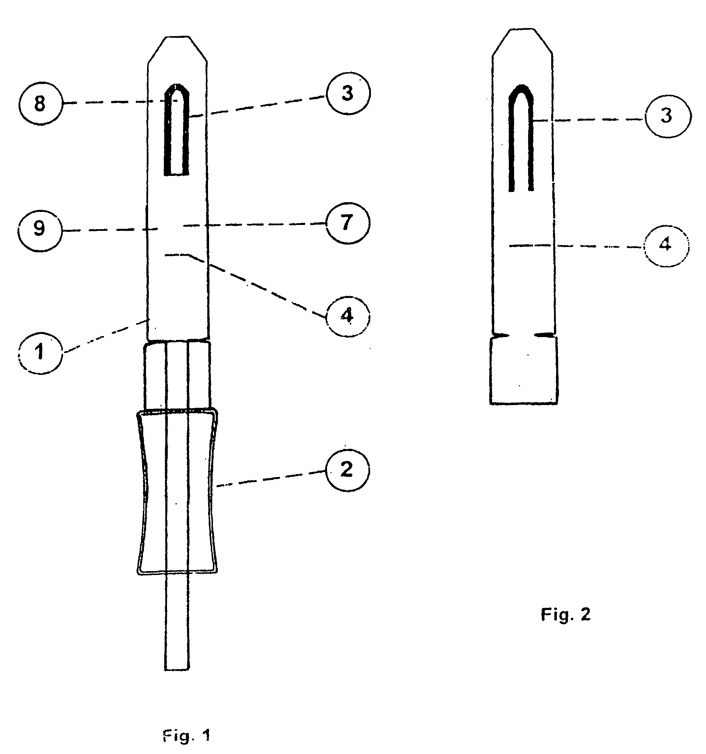

Regarding the present invention with reference to the accompanying figures 1 and 2 where the needle assembly (1) comprises a hub (2) where the cannula (7) is fixed to the hub (2) with the help of a suitable adhesive. The bevel end of a cannula (8) is siliconised and cured for the required period of time. The bevel protection sleeve (3) are washed ultrasonically and dried at a temperature of 40°-60° C. The bevel protection sleeve (3) is then capped on bevel end (8) of the cannula (7) already fixed to the hub, taking care that the closed tip of the bevel protection sleeve is not penetrated by the cannula bevel. The soft polyvinyl chloride needle cap is then (9) is then slid over the bevel protection sleeve (3) on the cannula once again taking care that undue pressure does not come on to the sleeve so that the cannula bevel (8) does not penetrate into the closed tip of a bevel protection sleeve. The cap is finally sealed air tight on to the hub (2) with the help of a adhesive making the needle assembly (1) ready for use.

An inbuilt barrier (4) is provided at one third to one fourth of the length of the needle cover (9) from the open end to facilitate preventing the sleeve (3) to come out when the cannula is taken out. Thus the sharp edge of the cannula is always protected.

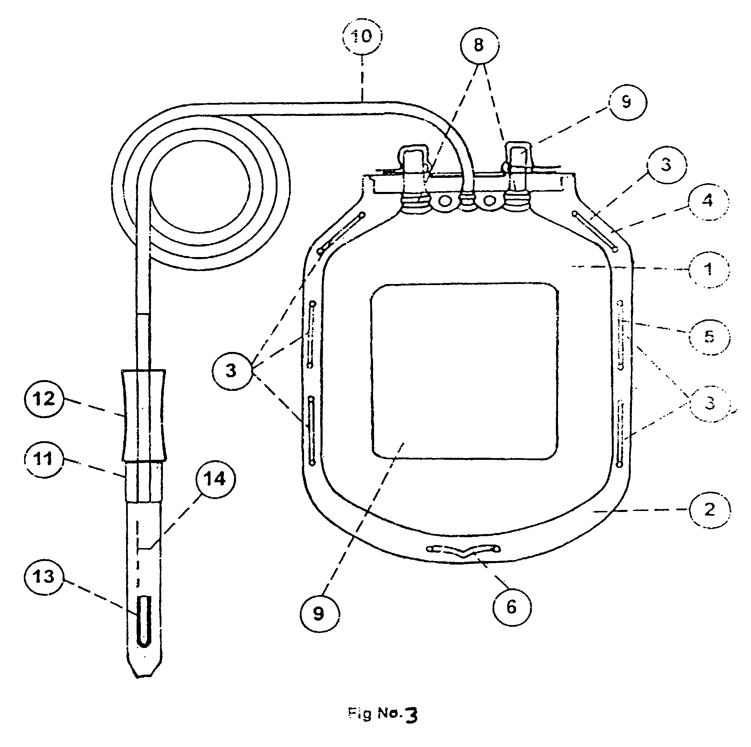

The figure 3 shows the parenteral solution container in its elevational view wherein the parenteral solution container (1) is made of soft polyvinyl chloride sheet having a novel composition as explained above. The parenteral solution container (1) has a peripheral seal (2) having tear seals (3) of desired width and size, located symmetrically in the side walls peripheral seal (2). The inside width (5) of the peripheral seal (2) around the notches is more than that the outer width (4) of the peripheral seal (2) around the tear seals (3) which gives extra strength to the healing and peripheral seal of the parenteral solution container. A hanger notch (6) in the form of tear seal provided in the bottom of the peripheral seal of the said parenteral solution container (1) to hang vertically down. A tamper proof non-peelable label is radio frequency sealed to the soft polyvinyl chloride sheet having also a temperature and time sensitive mark.

The ribs (8) are provided around the port of tubes (9) sealing area to give extra strength to the said parenteral solution container (1), eliminating chances of collapse of port tubing. The parenteral solution container (1) is connected to the needle assembly (11). The needle assembly as shown has a novel bevel protection sleeve (13), a barrier (14) and a hub (12).

The present invention can be more clearly understood with reference to the following examples. However, these examples do not act as undue limitation on the broad scope of the invention specifically with reference to the colour combination.

### EXAMPLES

### Example 1

Green colour changes to purple when subjected to steam autoclaving at 121° C for a period of 20 minutes.

### Example 2

Blue changes to Brown/Red/ Violet when subjected to steam autoclaving at 121° C for a period of 20 minutes.

### Example 3

White changes to Red when subjected to steam autoclaving at 121° C for a period of 20 minutes.

### Example 4

Yellow changes to Brown/Red when subjected to steam autoclaving at 121° C for a period of 20 minutes.

### Example 5

A medical grade paper of 65gsm is taken having its one side uncoated with the mat surface. A plastic film heat and radio frequency sealable of 30-40 micron supported on a carrier in the form of roll having same width as that of the paper is taken. The paper roll and the polyester film roll are mounted on a laminating and slitting machine. The uncoated side of the paper is laminated to the film suing a temperature 110-120° C on a laminating roll and using a pressure roll. The carrier film is then reparted. The laminated paper is then slit into widths suitable for printing. Printing contents of the label are printed using required number of colours. The ink to be used is pigmented grade ink. The subsequent printing head is used to make a mark of any suitable shape and size on the label using time and temperature sensitive ink. These labels are punched into single label. The labels are then radio frequency sealed on soft PVC film of the blood bag or any other medical bags.

## Claims

1. A flexible collapsible blood bag capable of being blow molded and auto claved from a soft polymeric film formed from a composition by radio frequency sealing the sides of the polymeric film comprising
a needle assembly (11) fixed at a PVC tubing provided at the sides of the blood bag for transfusion and collection of the blood, the needle assembly (11) comprising a needle cover (9) made from a soft polyvinyl chloride, having its one end closed and the other end opened, a hub (2) fitted to said needle cover (9) and a cannula (7) fixed to said hub (2)
a pair of port tubes in the upper peripheral seal of the blood bag,
a centrally located hanging means provided in the peripheral seal of the bag and
a series of symmetrical notches provided in the outer peripheral seal of the blood bag
**characterized by**
a pair of ribs (8) provided around the port tubes sealing area to give extra strength to the blood bag and to eliminate chances of collapse of the port tubes and
a non peelable tamperproof label or sticker radio frequency sealed on the blood bag which label comprising laminated paper having on its outer surface side contents printed by number of different or same colors and temperature and/or time sensitive means capable of changing color when subjected to steam autoclaving, wherein
the hub (2) of the needle assembly (11) in airtight fitted to the needle cover (9) and the needle assembly (11) comprises a bevel protection sleeve (3) capping on the bevel end of the cannula (7) wherein a barrier (4) is provided near the open end of said needle cover (9) the inner diameter of the barrier (4) being such that it prevents the bevel protection sleeve (3) from coming out when the cannula (7) is taken out from the needle cover (9).

2. A flexible, collapsible blood bag capable of being blow molded and auto claved according to claim 1, wherein the said composition comprises
| | |
|---|---|
| Polyvinyl chloride Resin | 100 parts per weight |
| Phthalate Plasticizer | 15 - 25 phr |
| Soyabean oil | 10 - 15 phr |
| Adipate | 15 - 25 phr |
| Stabilizer | 1.5 - 3.0 phr |
| Chilator | 0.5 - 1.5 phr |
| Lubricant | 0.25 - 0.75 phr. |

3. A flexible, collapsible blood bag capable of being blow molded and auto claved according to claim 2, wherein the soyabean oil is an epoxidised soyabean oil

4. A flexible, collapsible blood bag capable of being blow molded and auto claved according to claim 2, wherein the phthalate plasticizer is dioctyl phthalate plasticizer.

5. A flexible, collapsible blood bag capable of being blow molded and auto claved according to claim 2, wherein the adipate used is dioctyl adipate.

6. A flexible, collapsible blood bag capable of being blow molded and auto claved according to claim 2, wherein the stabilizer is calcium zinc heat stabilizer.

7. A flexible, collapsible blood bag capable of being blow molded and auto claved according to claim 2, wherein the chilator is organic phosphite chilator.

8. A flexible, collapsible blood bag capable of being blow molded and auto claved according to claim 2, wherein the lubricant is calcium stearate.

9. A flexible, collapsible blood bag capable of being blow molded and auto claved according to the claim 1, wherein the PVC tubing is formed from a polymeric film formed by a composition comprising
| | |
|---|---|
| Polyvinyl chloride Resin | 100 parts per weight |
| Phthalate Plasticizer | 15 - 25 phr |
| Soyabean oil | 10 - 15 phr |
| Adipate | 15 - 25 phr |
| Stabilizer | 1.5 - 3,0 phr |
| Chilator | 0,5 - 1,5 phr |
| Lubricant | 0,25 - 0,75 phr |

10. A flexible, collapsible blood bag capable of being blow molded and auto claved according to claim 1, wherein the said bevel protection sleeve (3) is made of rubber preferably medical grade rubber.

11. A flexible, collapsible blood bag capable of being blow molded and auto claved according to claim 1 wherein the hanging means is a hanger notch (6) in the form of tear seal provided at the bottom of the peripheral seal of the said blood bag, said hanger notch (6) forming angles to the center to enable the said blood bag to hang vertically down at the time of blood transfusion.

12. A flexible, collapsible blood bag capable of being blow molded and auto claved according to claim 11, wherein the wall thickness of the peripheral seal at the outer side of said tear seal is less than the wall thickness of the peripheral seal of the inner side of the said tear seal.

13. A flexible, collapsible blood bag capable of being blow molded and auto claved according to claim 11, wherein more than two tear seals in the walls of the peripheral seal preferably three on each sides are provided.

14. A flexible, collapsible blood bag capable of being blow molded and auto claved according to claim 1, wherein the said inbuilt barrier (4) is provided at the one third to one fourth of the length of the said needle cover (9) from the open end.

15. A flexible, collapsible blood bag capable of being blow molded and auto claved according to preceding claim 1, wherein the outer diameter of the said bevel protection sleeve (3) at its open end is less than the inner diameter of the said barrier (4) and the length of the bevel protection sleeve (3) prevents the solution contained in the bag to come to needle cover (9).

## Patentansprüche

1. Flexibler zusammenfallender Blutbeutel, der durch Blasformen und Behandeln im Autoclaven aus einer weichen Polymerfolie aus einer Zusammensetzung gebildet werden kann, indem die Seiten der Polymerfolie hochfrequenzverschweißt werden, mit
einer Nadelanordnung (11), die an einem PVC-Schlauch befestigt ist, der an der Seite des Blutbeutels zur Transfusion und Aufnahme des Blutes vorgesehen ist, welche Nadelanordnung (11) eine Nadelabdeckung (9) aus einem weichen Polyvinylchlorid, deren eines Ende geschlossen und deren anderes Ende offen ist, einen nabenartigen Körper (2), der an die Nadelabdeckung (9) gepasst ist, und eine Kanüle (7) umfasst, die am nabenartigen Körper (2) befestigt ist,
zwei Anschlussrohren in der oberen Umfangsdichtung des Blutbeutels,
einer zentral angeordneten Aufhängeeinrichtung, die in der Umfangsdichtung des Beutels vorgesehen ist, und
einer Reihe von symmetrischen Kerben, die in der äußeren Umfangsdichtung des Blutbeutels vorgesehen sind,
**gekennzeichnet durch**
zwei Rippen (8), die um den Anschlussrohrdichtungsbereich herum vorgesehen sind, um dem Blutbeutel eine zusätzliche Festigkeit zu geben und die Gefahr auszuschließen, dass die Anschlussrohre zusammenfallen, und
ein nicht ablösbares, gegen einen unbefugten Eingriff gesichertes Etikett oder einen entsprechender Aufkleber, das/der **durch** Hochfrequenzschweißen auf den Blutbeutel aufgebracht ist, welches Etikett ein Schichtpapier umfasst, das an seiner Außenflächenseite den Inhalt in Form von aufgedruckten Zahlen in verschiedenen oder gleichen Farben und temperatur- und/oder zeitempfindliche Einrichtungen aufweist, die ihre Farbe ändern können, wenn sie einer Dampfautoclavenbehandlung unterworfen werden,
wobei der nabenartige Körper (2) der Nadelanordnung (11) luftdicht an die Nadelabdeckung (9) gepasst ist und die Nadelanordnung (11) eine Zuschärfungsschutzhülse (3) umfasst, die als Kappe auf dem Zuschärfungsende der Kanüle (7) sitzt, wobei eine Sperre (4) in der Nähe des offenen Endes der Nadelabdeckung (9) vorgesehen ist, deren Innendurchmesser so gewählt ist, dass sie einen Abgang der Zuschärfungsschutzhülse (3) verhindert, wenn die Kanüle (7) aus der Nadelabdeckung (9) genommen wird.

2. Flexibler zusammenfallender Blutbeutel, der durch Blasformen und Behandeln im Autoclaven gebildet werden kann, nach Anspruch 1, bei dem die Zusammensetzung
| | |
|---|---|
| Polyvinylchloridharz | 100 Gewichtsteile |
| Phthalatweichmacher | 15 - 25 Teile pro 100 Teile Harz |
| Sojaöl | 10 - 15 Teile pro 100 Teile Harz |
| Adipat | 15 - 25 Teile pro 100 Teile Harz |
| Stabilisator | 1,5-3,0 Teile pro 100 Teile Harz |
| Chelatbildner | 0,5-1,5 Teile pro 100 Teile Harz |
| Gleitmittel | 0,25-0,75 Teile pro 100 Teile Harz |
umfasst.

3. Flexibler zusammenfallender Blutbeutel, der durch Blasformen und Behandeln im Autoclaven gebildet werden kann, nach Anspruch 2, bei dem das Sojaöl ein epoxidiertes Sojaöl ist.

4. Flexibler zusammenfallender Blutbeutel, der durch Blasformen und Behandeln im Autoclaven gebildet werden kann, nach Anspruch 2, bei dem der Phthalatweichmacher ein Dioktylphthalat-Weichmacher ist.

5. Flexibler zusammenfallender Blutbeutel, der durch Blasformen und Behandeln im Autoclaven gebildet werden kann, nach Anspruch 2, bei dem das benutzte Adipat ein Dioktyladipat ist.

6. Flexibler zusammenfallender Blutbeutel, der durch Blasformen und Behandeln im Autoclaven gebildet werden kann, nach Anspruch 2, bei dem der Stabilisator ein Kalziumzink-Wärmestabilisator ist.

7. Flexibler zusammenfallender Blutbeutel, der durch Blasformen und Behandeln im Autoclaven gebildet werden kann, nach Anspruch 2, bei dem der Chelatbildner ein organischer Phosphitchelatbildner ist.

8. Flexibler zusammenfallender Blutbeutel, der durch Blasformen und Behandeln im Autoclaven gebildet werden kann, nach Anspruch 2, bei dem das Gleitmittel Kalziumstearat ist.

9. Flexibler zusammenfallender Blutbeutel, der durch Blasformen und Behandeln im Autoclaven gebildet werden kann, nach Anspruch 1, bei dem der PVC-Schlauch aus einer Polymerfolie gebildet ist, die aus einer Zusammensetzung gebildet ist, die
| | |
|---|---|
| Polyvinylchloridharz | 100 Gewichtsteile |
| Phthalatweichmacher | 15 - 25 Teile pro 100 Teile Harz |
| Sojaöl | 10 - 15 Teile pro 100 Teile Harz |
| Adipat | 15 - 25 Teile pro 100 Teile Harz |
| Stabilisator | 1,5-3,0 Teile pro 100 Teile Harz |
| Chelatbildner | 0,5-1,5 Teile pro 100 Teile Harz |
| Gleitmittel | 0,25 - 0,75 Teile pro 100 Teile Harz |
umfasst.

10. Flexibler zusammenfallender Blutbeutel, der durch Blasformen und Behandeln im Autoclaven gebildet werden kann, nach Anspruch 1, bei dem die Zuschärfungsschutzhülse (3) aus Gummi, vorzugsweise Medizingummi, besteht.

11. Flexibler zusammenfallender Blutbeutel, der durch Blasformen und Behandeln im Autoclaven gebildet werden kann, nach Anspruch 1, bei dem die Aufhängeeinrichtung eine Aufhängenut (6) in Form einer Reißdichtung ist, die am unteren Teil der Umfangsdichtung des Blutbeutels vorgesehen ist, welche Aufhängenut (6) zur Mitte Winkel bildet, um den Blutbeutel vertikal nach unten während der Bluttransfusion aufhängen zu können.

12. Flexibler zusammenfallender Blutbeutel, der durch Blasformen und Behandeln im Autoclaven gebildet werden kann, nach Anspruch 11, bei dem die Wandstärke der Umfangsdichtung an der Außenseite der Reißdichtung kleiner als die Wandstärke der Umfangsdichtung an der Innenseite der Reißdichtung ist.

13. Flexibler zusammenfallender Blutbeutel, der durch Blasformen und Behandeln im Autoclaven gebildet werden kann, nach Anspruch 11, bei dem mehr als zwei Reißdichtungen in den Wänden der Umfangsdichtung, vorzugsweise drei auf jeder Seite, vorgesehen sind.

14. Flexibler zusammenfallender Blutbeutel, der durch Blasformen und Behandeln im Autoclaven gebildet werden kann, nach Anspruch 1, bei dem die eingebaute Sperre (4) auf einem Drittel bis einem Viertel der Länge der Nadelabdeckung (9) vom offenen Ende aus vorgesehen ist.

15. Flexibler zusammenfallender Blutbeutel, der durch Blasformen und Behandeln im Autoclaven gebildet werden kann, nach Anspruch 1, bei dem der Außendurchmesser der Zuschärfungsschutzhülse (3) an ihrem offenen Ende kleiner als der Innendurchmesser der Sperre (4) ist, und die Länge der Zuschärfungsschutzhülse (3) verhindert, dass die im Beutel enthaltene Lösung die Nadelabdeckung (9) erreicht.

## Revendications

1. Poche de sang flexible et repliable capable d'être moulée-soufflée et autoclavée à partir d'un film polymère mou formée à partir d'une composition en soudant par radio fréquence les bords du film polymère comprenant :
un bloc aiguille (11) fixé à une tubulure en PVC située au niveau des bords de la poche de sang pour la transfusion et le prélèvement du sang, le bloc aiguille (11) comprenant un étui protecteur d'aiguille (9) fabriqué à partir d'un polychlorure de vinyle mou, ayant l'une de ses extrémités fermée et l'autre extrémité ouverte, une embase (2) montée sur ledit étui protecteur d'aiguille (9) et une canule (7) fixée à ladite embase (2),
deux tubes à embouchure dans la soudure périphérique supérieure de la poche de sang,
un moyen de suspension placé au milieu situé dans la soudure périphérique de la poche de sang et
une série d'encoches symétriques situées dans la soudure périphérique externe de la poche de sang
**caractérisée par**
deux nervures (8) situées autour de la zone de soudure des tubes à embouchure pour donner une résistance supplémentaire à la poche de sang et pour éliminer les risques d'affaissement des tubes à embouchure et
une étiquette ou un autocollant d'inviolabilité non pelable soudé par radio fréquence sur la poche de sang laquelle étiquette comprenant du papier laminé ayant sur sa surface externe les contenus imprimés par un certain nombre de couleurs différentes ou identiques et des moyens sensibles au temps et/ou à la température capables de changer de couleur lorsqu'ils sont soumis à l'autoclavage à vapeur, dans laquelle
l'embase (2) du bloc aiguille (11) est fixée de façon hermétique à l'étui protecteur d'aiguille (9) et le bloc aiguille (11) comprend un manchon de protection du biseau (3) couvrant l'extrémité du biseau de la canule (7) dans lequel une barrière (4) est située près de l'extrémité ouverte dudit étui protecteur d'aiguille (9) le diamètre intérieur de la barrière (4) étant tel qu'il empêche le manchon de protection du biseau (3) de sortir lorsque la canule (7) est retirée de l'étui protecteur d'aiguille (9).

2. Poche de sang flexible et repliable capable d'être moulée-soufflée et autoclavée selon la revendication 1, dans laquelle ladite composition comprend :
| | |
|---|---|
| Résine de polychlorure de vinyle | 100 parties par poids |
| Plastifiant à base de phtalate | 15 - 25 pcc* |
| Huile de soja | 10 - 15 pcc* |
| Adipate | 15 - 25 pcc* |
| Stabilisant | 1,5 - 3,0 pcc* |
| Chélateur | 0,5 - 1,5 pcc* |
| Lubrifiant | 0,25 - 0,75 pcc* |
| | |
|---|---|
| * parties pour cent parties de caoutchouc | |

3. Poche de sang flexible et repliable capable d'être moulée-soufflée et autoclavée selon la revendication 2, dans laquelle l'huile de soja est une huile de soja époxyde.

4. Poche de sang flexible et repliable capable d'être moulée-soufflée et autoclavée selon la revendication 2, dans laquelle le plastifiant à base de phtalate est un plastifiant de phtalate de dioctyle.

5. Poche de sang flexible et repliable capable d'être moulée-soufflée et autoclavée selon la revendication 2, dans laquelle l'adipate utilisé est l'adipate de dioctyle.

6. Poche de sang flexible et repliable capable d'être moulée-soufflée et autoclavée selon la revendication 2, dans laquelle le stabilisant est un stabilisant thermique zinc calcium.

7. Poche de sang flexible et repliable capable d'être moulée-soufflée et autoclavée selon la revendication 2, dans laquelle le chélateur est un chélateur à base de phosphite organique.

8. Poche de sang flexible et repliable capable d'être moulée-soufflée et autoclavée selon la revendication 2, dans laquelle le lubrifiant est du stéarate de calcium.

9. Poche de sang flexible et repliable capable d'être moulée-soufflée et autoclavée selon la revendication 1, dans laquelle la tubulure en PVC est formée à partir d'un film polymère formé par une composition comprenant :
| | |
|---|---|
| Résine de polychlorure de vinyle | 100 parties par poids |
| Plastifiant à base de phtalate | 15 - 25 pcc* |
| Huile de soja | 10 - 15 pcc* |
| Adipate | 15 - 25 pcc* |
| Stabilisant | 1,5 - 3,0 pcc* |
| Chélateur | 0,5 - 1,5 pcc* |
| Lubrifiant | 0,25 - 0,75 pcc* |

10. Poche de sang flexible et repliable capable d'être moulée-soufflée et autoclavée selon la revendication 1, dans laquelle ledit manchon de. protection du biseau (3) est en caoutchouc de préférence du caoutchouc de qualité médicale.

11. Poche de sang flexible et repliable capable d'être moulée-soufflée et autoclavée selon la revendication 1, dans laquelle le moyen de suspension est une encoche de suspension (6) sous la forme d'un joint antirupture situé en bas de la soudure périphérique de ladite poche de sang, ladite-encoche de suspension (6) formant des angles par rapport au centre pour permettre à ladite poche de sang d'être suspendue verticalement vers le bas au moment de la transfusion sanguine.

12. Poché de sang flexible et repliable capable d'être moulée-soufflée et autoclavée selon la revendication 11, dans laquelle l'épaisseur de la paroi de la soudure périphérique au niveau du bord externe dudit joint antirupture est inférieure à l'épaisseur de la paroi de la soudure périphérique du bord interne dudit joint antirupture.

13. Poche de sang flexible et repliable capable d'être moulée-soufflée et autoclavée selon la revendication 11, dans laquelle plus de deux joints antiruptures dans les parois de la soudure périphérique de préférence trois sur chaque bord sont proposés.

14. Poche de sang flexible et repliable capable d'être moulée-soufflée et autoclavée selon la revendication 1, dans laquelle ladite barrière incorporée (4) est située à un niveau compris entre le tiers et le quart de la longueur dudit étui protecteur d'aiguille (9) à partir de l'extrémité ouverte.

15. Poche de sang flexible et repliable capable d'être moulée-soufflée et autoclavée selon la revendication 1, dans laquelle le diamètre extérieur dudit manchon de protection du biseau (3) à son extrémité ouverte est inférieur au diamètre intérieur de ladite barrière (4) et la longueur du manchon de protection du biseau (3) empêche la solution contenue dans la poche d'arriver à l'étui protecteur d'aiguille (9).
